# EUROPÄISCHE PATENTSCHRIFT

(11) **EP 2 317 962 B1**
(45) Veröffentlichungstag und Bekanntmachung des Hinweises auf die Patenterteilung: **27.11.2013**
(21) Anmeldenummer: 08773562.7
(22) Anmeldetag: 20.06.2008
(51) Int. Cl.: A61F 9/009, A61F 9/01

(54) **VORRICHTUNG ZUM SCHNEIDEN EINES GEWEBETEILS MIT FOKUSSIERTER LASERSTRAHLUNG**
DEVICE FOR CUTTING A TISSUE PART WITH FOCUSSED LASER RADIATION
DISPOSITIF DE DÉCOUPE D'UN FRAGMENT DE TISSU À L'AIDE D'UN FAISCEAU LASER FOCALISÉ

(43) Veröffentlichungstag der Anmeldung: 11.05.2011
(73) Patentinhaber: WaveLight GmbH, 91058 Erlangen (DE)
(72) Erfinder: DONITZKY, Christof, 90542 Eckental (DE); WÜLLNER, Christian, 91094 Bräunigshof (DE)
(74) Vertreter: von Hellfeld, Axel
(86) Internationale Anmeldenummer: PCT/EP2008/005012
(87) Internationale Veröffentlichungsnummer: WO 2009/152838

(56) Entgegenhaltungen:
- WO-A-2005/048896
- WO-A-2008/008330
- WO-A-2008/072092
- DE-A1- 3 612 287
- DE-A1-102006 056 711
- DE-B3- 10 330 821
- US-A- 5 507 741
- US-A- 5 549 632
- US-A- 5 807 380

## Beschreibung

Die Erfindung betrifft eine Vorrichtung zum Schneiden eines Gewebeteils aus einem Gewebe mit fokussierter Laserstrahlung. Insbesondere betrifft die Vorrichtung das Schneiden eines sog. Flaps (Deckelchens) aus der Kornea eines Auges bei der refraktiven Chirurgie, insbesondere LASIK. Nachfolgend wird die Erfindung mit Blick auf die refraktive Chirurgie, insbesondere LASIK näher erläutert. Daneben ist die Erfindung aber auch einsetzbar allgemein zum schneiden eines Gewebeteils aus einem Gewebe mittels fokussierter Laserstrahlung.

Es ist bekannt, bei der LASIK, also der inzwischen allgemein bekannten Korrektur der optischen Abbildungseigenschaften der Kornea mittels Laserstrahlung, zunächst ein sog. Flap (Deckelchen) aus dem vorderen Bereich der Kornea herauszuschneiden, wobei ein Abschnitt des Flaps wie ein Gelenk ("hinge": SCharnier) mit der Kornea verbunden bleibt, so dass das Flap für die anschließende Ablation von Komeagewebe mittels Laserstrahlung zur Seite geklappt werden kann. Nach Durchführung der Ablation (Gewebeabtrag) wird das Flap zurückgeklappt und es erfolgt eine relative schnelle Heilung unter weitgehender Unversehrtheit der Korneaoberfläche.

Der Stand der Technik kennt hauptsächlich zwei unterschiedliche Techniken für die Erzeugung des Flap.

Zum Einen wird für das Schneiden des Flaps ein mechanisches sog. Mikrocheratom eingesetzt, welches mit einer schnell oszillierenden Schneide von der Seite her in die Kornea einschneidet. Dabei wird ein sog. Saugring auf das Auge aufgesetzt, der mittels Vakuum das Auge fixiert. Das ist insoweit Stand der Technik. So ein Gerät ist beispielsweise in US 5 807 380 beschrieben.

Zum Anderen wird zunehmend auch für das Schneiden des Flaps Laserstrahlung eingesetzt, zur Zeit fokussierte Laserstrahlung mit Pulslängen im Femtosekundenbereich. Die Strahlung wird dabei unter der Vorderfläche der Kornea im Inneren des Gewebes fokussiert und die Fokuspunkte werden in der gewünschten Fläche so positioniert, dass im Ergebnis ein Flap aus der Kornea herausgeschnitten wird. Dies ist als Femto-LASIK im Stand der Technik gut bekannt.

Die Femto-LASIK kennt zur Zeit insbesondere zwei Ausführungsvarianten:
Gemäß einer ersten Ausführungsform wird ein separates System aus Saugring und Lidsperrung zur lateralen Fixierung des Auges eingesetzt. Eine planare (ebene) optische Platte wird zur Einebnung der Kornea aufgedrückt. Das Stroma wird flächig in der Tiefe in der oben beschriebenen Weise geschnitten. Der Randschnitt (also die Positionierung der Fokuspunkte im Randbereich des Flaps) erfolgt dabei durch Positionierung der Fokuspunkte aus der Ebene heraus bis hin zur Kornea-Oberfläche. Dabei gelangen die Fokuspunkte in die genannte planare optische Platte hinein, welche dadurch zerstört wird und somit einen Einmalartikel darstellt.

Gemäß einer anderen Ausführungsform der Femto-LASIK wird der planare stromale Schnitt ebenfalls unter Einsatz eines Saugrings mit einer Applanation unter Ansaugen des Auges durchgeführt. Dabei wird die Schnittiefe durch eine für die Laserwellenlänge transparente Kunststofffolie definiert, welche zwischen der Applanationsplatte und der Kornea liegt. Auch dabei ist die Kunststofffolie ein Einmalartikel. Eine besondere Ausgestaltung des Schnittrands ist dabei nicht vorgesehen.

Die Erfindung hat zum Ziel, eine Vorrichtung bereitzustellen, mit der in einfacher Weise mit fokussierter Laserstrahlung ein Gewebeteil, insbesondere ein Flap, aus einem Gewebe, insbesondere der Komea, schneidbar ist. Dabei soll insbesondere die Operationsdauer verkürzt und die für den Schnitt in das Auge eingebrachte Energie verringert werden.

Hierzu stellt die Efindung eine Vorrichtung gemäß Anspruch 1 bereit zum Schneiden eines Gewebeteils aus einem Gewebe mittels fokussierter Laserstrahlung.

Gemäß der Erfindung kann zum Beispiel bei der Femto-LASIK für jeden gewünschten Flapdurchmesser und für jede gewünschte Geometrie des Flaps ein "maßgeschneiderter" für die Laserstrahlung undurchlässiger Körper im vorstehenden Sinn ausgewählt und mit dem Saugring eingesetzt werden. Dabei kann der genannte, für die Laserstrahlung undurchlässige Körper fest mit dem Saugring verbunden sein, so dass für die jeweils gewünschte Form des Flaps ein spezifischer Saugring hergenommen wird, oder der für die Laserstrahlung undurchlässige Körper als Austauschteil mit dem Saugring lösbar verbunden.

Die "Applanationsplatte" im Sinne der vorliegenden Erfindung muss nicht notwendigerweise planar (eben) sein, sondern kann auch eine z.B. sphärische Krümmung aufweisen, je nach dem gewünschten Schnitt.

Bevorzugt ist der genannte für Laserstrahlung undurchlässige Körper ringförmig.

Eine andere Ausgestaltung sieht vor, dass der für die Laserstrahlung undurchlässige Körper entweder die Laserstrahlung teilweise absorbiert oder ganz absorbiert.

Eine andere Ausgestaltung der Erfindung sieht vor, dass der für Laserstrahlung undurchlässige Körper auf einer Kontaktplatte aufliegt, die mit einer Dichtfläche in Anlage an das Gewebe bringbar ist.

Eine Vorrichtung, die die Merkmale des Oberbegriffs des Anspruchs 1 beinhaltet ist aus WO 2008/008 330 A2 bekannt, welche aber, unter anderem, nicht dafür geeignet ist, Gewebeteile aus einem gewebe zu schneiden.

Nachfolgend werden Ausführungsbeispiele der Erfindung anhand der Zeichnung näher erläutert. Es zeigt:
Fig. 1A-1C schematisch die Erzeugung eines Flaps bei der Femto-LASIK;
Fig. 2 schematisch ein Ausführungsbeispiel einer Vorrichtung zum Schneiden eines Gewebeteils aus Gewebe gemäß der Erfindung;
Fig. 3 einen Ausschnitt von Fig. 2 in vergrößertem Maßstab; und
Fig. 4 eine Draufsicht auf einen für Laserstrahlung undurchlässigen Körper.

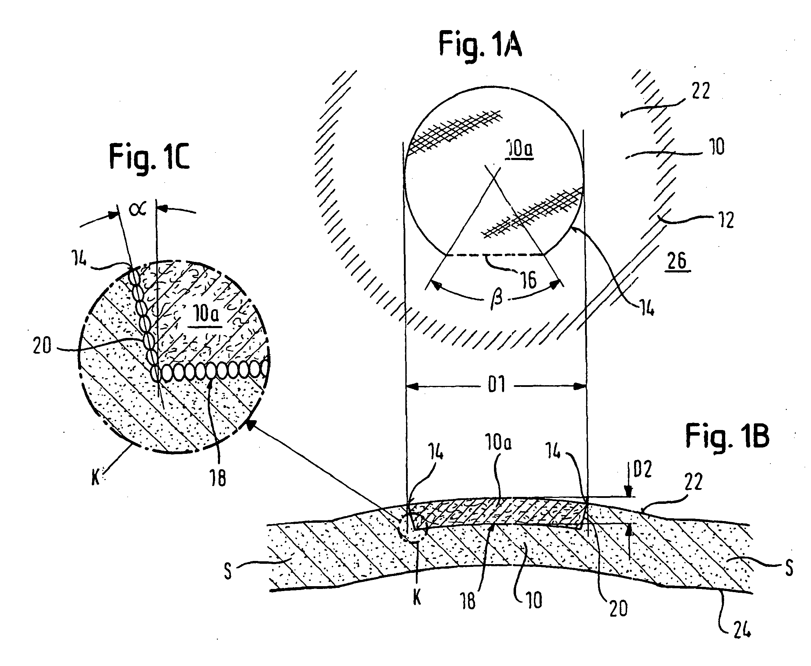

Die Figuren 1A, 1B und 1C zeigen schematisch die Verhältnisse beim Schneiden eines Flap 10a in einer Kornea 10 unter Verwendung von fokussierter Strahlung mit Pulslängen im Femtosekundenbereich. Die verwendete Laserwellenlänge entspricht dem Stand der Technik.

Fig. 1A zeigt eine Draufsicht auf die Kornea 10, deren Limbus 12, den Rand 14 des Flaps 10a und die Sklera 26. In einem Gelenkteil 16 ("hinge"; Scharnier) bleibt das geschnittene Flap 10a mit der Kornea 10 verbunden, so dass es in an sich bekannter Weise aufklappbar ist. Fig. 1B zeigt die Laserfokusebene 18, also diejenige Ebene, in welcher die Fokuspunkte der Laserstrahlung so positioniert werden, dass insgesamt ein Schnitt entsteht. Dabei ist der hier verwendete Begriff "Ebene" in der Fachsprache nicht als unbedingt planar zu verstehen, sondern umfasst auch gekrümmte Gestaltungen der Fläche der Fokuspunkte, wie zum Beispiel der Schnitt gemäß Fig. 1B zeigt.

Der Kreisausschnitt K gemäß Fig. 1B ist in Fig. 1C in vergrößertem Maßstab dargestellt. Danach wird die Fläche, in der die Laserfokuspunkte platziert werden im Randbereich in einem Seitenschnitt 20 nach oben gezogen. Dort, wo die Schnittebene der Fokuspunkte die Vorderfläche 22 der Kornea 10 schneidet, befindet sich der Rand 14 des geschnittenen Flaps. Fig. 1B zeigt auch den Durchmesser D1 und die Stärke D2 des Flaps, und die Hinterfläche 24 der Kornea 10.

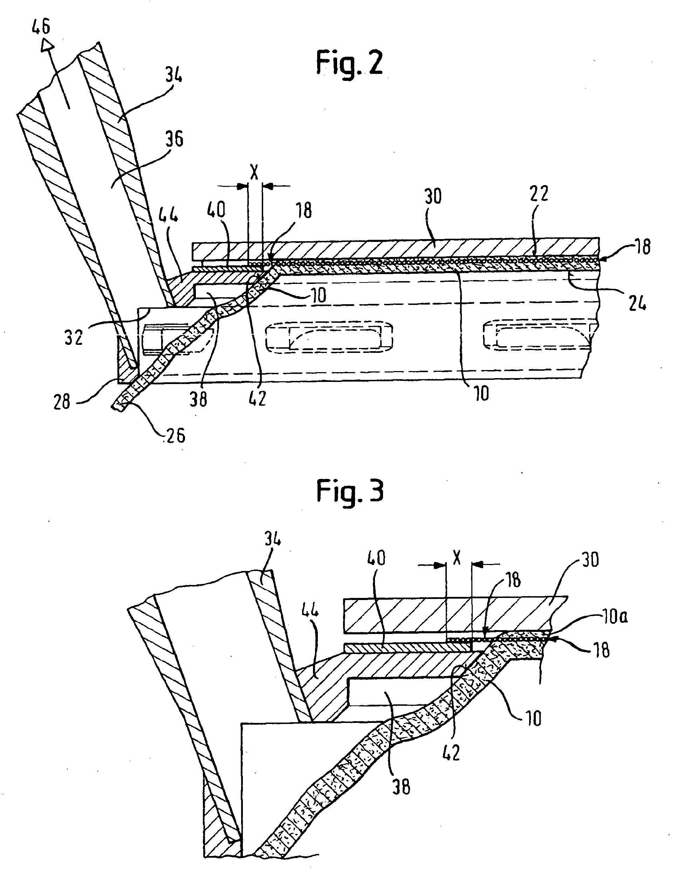

Der Seitenschnitt 20 und die entsprechende Führung der Fokuspunkte der Laserstrahlung ist aufwendig und kompliziert, was letztlich auch Auswirkungen auf die Wahrscheinlichkeit von nicht perfekten Schnitten hat. Auch ist der Seitenschnitt am Rand 14 zeitaufwendig und bedingt eine relativ hohe Gesamtenergie, die in das Auge eingebracht wird. Beides wird mit einer Vorrichtung gemäß den Fig. 2 bis 4 verbessert.

Fig. 2 zeigt einen Saugring 28 mit einer Applanationsplatte 30, die auf die Vorderfläche 22 der Kornea 10 aufgedrückt wird. Wie oben bereits ausgeführt ist, muss die Applanationsplatte 30 nicht unbedingt planar (eben) sein, sondern kann auch gekrümmt sein, zum Beispiel sphärisch.

Der Saugring 28 hat in an sich bekannter Weise Öffnungen 32 zur Erzeugung eines wirksamen aber nicht zu hohen Unterdrucks im Raum 38 derart, dass das Auge lateral im Saugring 28 fixiert ist. Der Saugring 28 weist weiterhin in an sich bekannter Weise einen Stutzen 34 auf, in dem ein Kanal 36 zu einer (nicht gezeigten) Vakuumpumpe führt, die durch das Bezugszeichen 46 angedeutet ist.

Der Saugring 28 weist eine Kontaktplatte 44 auf, die mit einer Dichtfläche 42 an der Vorderfläche 22 der Kornea 10 anliegt, so dass der Raum 38 gegen die Kornea abgedichtet ist und in ihm gegenüber der äußeren Atmosphäre ein Unterdruck herrscht.

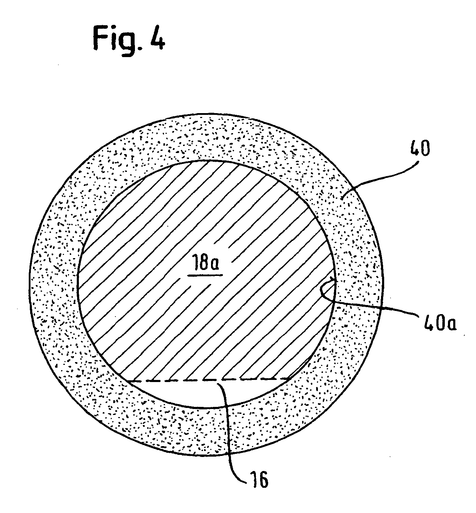

Die Fläche 18, in welcher die Laser-Fokuspunkte positioniert werden, ist in den Fig. 2 und 3 dargestellt. Diese Laserfokusebene ist beim gezeigten Ausführungsbeispiel vollständig planar. Dies gilt insbesondere für den Bereich am Rand 14 des Schnitts. Zur Definition des Schnittrands 14 dient ein Körper 40 der für die Laserstrahlung undurchlässig ist. Fig. 4 zeigt in Draufsicht ein Ausführungsbeispiel des für Laserstrahlung undurchlässigen Körpers 40. Der Körper 40 wird gemäß Fig. 2 auf die genannte Saugringkontaktplatte 44 aufgelegt. Er liegt unter der Applanationsplatte 30 und ist so gewählt, dass sein Innenrand 40A (vgl. Fig. 4) dem gewünschten Rand 14 des Flap 10a entspricht. Somit kann durch Auswahl der Form des Körpers 40 die Form des Flaps 10a bestimmt werden. Gemäß Fig. 4 werden die Laserpulse im Bereich 18a wirksam, d.h. der Laserschnitt erfolgt ausschließlich in der in Fig. 4 schraffiert gezeichneten Fläche 18a, so dass der Gelenkbereich 16 ungeschnitten bleibt. Ein abgewinkelter Seitenschnitt 20 mit dem Winkel α gemäß Fig. 1C ist hier nicht erforderlich. Vielmehr kann im Randbereich die Schnittführung planar bleiben. Fig. 2 zeigt den Bereich "X", in dem die Laserstrahlung ohne Nachteile für den gewünschten Schnitt über den für Laserstrahlung undurchlässigen Körper 40 geführt werden kann. Dies vereinfacht die Steuerung für den Laserstrahl.

Das Ausführungsbeispiel gemäß Fig. 2 zeigt eine im strengen Sinne planare Ebene 18 der Laserfokuspunkte, jedoch kann im Inneren des Schnitts, also außerhalb des Bereichs direkt um den Rand 14, die Schnittführung auch anders als eben sein, zum Beispiel sphärisch oder dergleichen.

Die Applanationsplatte 30 bleibt bei der Operation unbeschädigt. Die Dicke (vgl. Fig. 1B, Bezugszeichen D2) des Flaps kann durch Variation der Tiefe der Fokuspunkte im stromalen Gewebe an die gewünschte Schnittform angepasst werden. Eine Veränderung der Position der Laserfokuspunkte im Randbereich In z-Richtung (üblicherweise wird hier mit "z-Richtung" die Richtung senkrecht zur Applanationsplatte; also im Wesentlichen in Richtung der optischen Achse definiert) erübrigt sich im Randbereich. Dadurch kann auch die Operationsdauer deutlich verkürzt werden.

### Bezugszeichenliste

- 10: Kornea
- 10a: Flap
- 12: Limbus
- 14: Rand (des Flap)
- 16: Gelenkteil ("Scharnier")
- 18: Laserfokusebene
- 18a: Schnittbereich
- 20: Seitenschnitt
- 22: Vorderfläche (von 10)
- 24: Hinterfläche (von 10)
- 26: Sklera
- 28: Saugring
- 30: Applanationsplatte
- 32: Öffnungen
- 34: Stutzen
- 36: Kanal
- 38: Hohlraum (Vakuum)
- 40: strahlungsundurchlässiger Körper
- 40a: Innenrand
- 42: Dichtfläche
- 44: Saugring-Kontaktplatte
- 46: Vakuumpumpe
- D1: Durchmesser
- D2: Stärke
- *α*: Winkel
- *β*: Winkel
- K: Kreis
- X: Bereich
- S: Stroma

## Patentansprüche

1. Vorrichtung zum Schneiden eines Gewebeteils (10a) aus einem Gewebe (10) mittels fokussierter Laserstrahlung, folgendes aufweisend:
- eine Lasereinrichtung zur Erzeugung der Laserstrahlung,
- einen Saugring (28), der eine Dichtfläche (42) und eine Kontaktplatte (44) hat, die an eine Oberfläche (22) des Gewebes (10) anlegbar sind,
- Einrichtungen (34, 36, 46) zum Erzeugen eines Unterdrucks in einem Hohlraum (38), der durch Anlegen der Dichtfläche (42) an die Oberfläche (22) von der Oberfläche (22) des Gewebes (10) und dem Saugring begrenzt wird,
- eine Applanationsplatte (30), die zur Formung auf die Oberfläche des Gewebes (10) andrückbar ist,
- einen für die Laserstrahlung undurchlässigen Körper (40),
**dadurch gekennzeichnet, dass**
der Körper (40) mit dem Saugring (28) zusammenwirkt und durch Anlegen der Dichtfläche (42) an die Oberfläche (22) mit seinem Innenrand (40a) einen Rand (14) des zu schneidenden Gewebeteils (10a) definiert, wobei der Körper (40) auf der Kontaktplatte (44) des Saugrings (28), die mit der Dichtfläche (42) in Anlage an das Gewebe (10) bringbar ist, und unter der Applanationsplatte (30) liegt.

2. Vorrichtung nach Anspruch 1 **dadurch gekennzeichnet, dass** der für Laserstrahlung undurchlässige Körper (40) ringförmig ist.

3. Vorrichtung nach einem der Ansprüche 1 oder 2 **dadurch gekennzeichnet, dass** der für Laserstrahlung undurchlässige Körper (40) die Laserstrahlung zumindest teilweise absorbiert.

4. Vorrichtung nach einem der vorhergehenden Ansprüche **dadurch gekennzeichnet, dass** die Dichtfläche (42) in Anlage an die Cornea (10) eines Auges bringbar ist und dass durch Anlegen der Dichtfläche (42) an die Oberfläche (22) der Innenrand (40a) des für die Laserstrahlung undurchlässigen Körpers (40) den Rand eines aufklappbaren Deckelchens (10a) bei einer ophthalmologischen Operation definiert.

## Claims

1. Apparatus for cutting a tissue part (10a) out of a tissue (10) by means of focused laser radiation, comprising the following:
- a light generating device for generating the laser radiation;
- a suction ring (28) which has a sealing surface (42) and a contact plate (44) that are capable of being applied onto a surface (22) of the tissue (10),
- devices (34, 36, 46) for generating an underpressure in a cavity (38) that is, by applying the sealing surface (42) onto the surface (22), delimited by the surface (22) of the tissue (10) and by the suction ring, and
- an applanation plate (30) that is capable of being pressed against the surface of the tissue (10) for the purpose of shaping,
- a body (40) that is opaque to the laser radiation,
**characterised in**
**that** the body (40) interacts with the suction ring (28) and, by applying the sealing surface (42) on to the surface (22) with its inner edge (40a), defines an edge (14) of the tissue part (10a) to be cut, wherein the body (40) rests on the contact plate (44) of the suction ring (28) that with the sealing surface (42) is capable of being brought into contact with the tissue (10) and wherein the body (40) is placed below the applanation plate (30).

2. Apparatus according to Claim 1, **characterised in that** the body (40) that is opaque to laser radiation is annular.

3. Apparatus according to one of Claims 1 or 2, **characterised in that** the body (40) that is opaque to laser radiation at least partly absorbs the laser radiation.

4. Apparatus according to one of the preceding claims, **characterised in that** the sealing surface (42) is capable of being brought into contact with the cornea (10) of an eye and **in that**, by applying the sealing surface (42) on to the surface (22), the inner edge (40a) of the body (40) that is opaque to the laser radiation defines the edge of a flap (10a) that is capable of bring folded upwards in the course of an ophthalmological operation.

## Revendications

1. Dispositif de découpe d'un fragment de tissus (10a) dans des tissus (10), au moyen d'un rayonnement laser focalisé, comportant :
- un appareil laser destiné à générer le rayonnement laser,
- un anneau d'aspiration (28) ayant une surface d'étanchéité (42) et une plaque de contact (44) qui peuvent être appliquées sur une surface (22) des tissus (10),
- des installations (34, 36, 46) destinées à générer une dépression dans une cavité (38) qui, après application de la surface d'étanchéité (42) sur la surface (22), est délimitée par la surface (22) des tissus (10) et l'anneau d'aspiration,
- une plaque d'aplanation (30) pouvant être pressée sur la surface des tissus (10) pour assurer une mise en forme,
- un corps (40) imperméable au rayonnement laser,
**caractérisé en ce que**
le corps (40) interagit avec l'anneau d'aspiration (28), et par application de la surface d'étanchéité (42) sur la surface (22), définit avec son bord intérieur (40a) un bord (14) du fragment de tissus (10a) à découper, le corps (40) reposant sur la plaque de contact (44) de l'anneau d'aspiration (28), qui peut être appliquée sur les tissus (10) avec la surface d'étanchéité (42), et sous la plaque d'aplanation (30).

2. Dispositif selon la revendication 1, **caractérisé en ce que** le corps (40) imperméable au rayonnement laser est de forme annulaire.

3. Dispositif selon l'une des revendications 1 ou 2, **caractérisé en ce que** le corps (40) imperméable au rayonnement laser absorbe au moins partiellement le rayonnement laser.

4. Dispositif selon l'une des revendications précédentes, **caractérisé en ce que** la surface d'étanchéité (42) peut être appliquée sur la cornée (10) d'un oeil, et **en ce que** par application de la surface d'étanchéité (42) sur la surface (22), le bord intérieur (40a) du corps (40) imperméable au rayonnement laser définit le bord d'un petit couvercle (10a) pouvant être relevé, lors d'une opération ophtalmologique.
